(19) **Europäisches Patentamt European Patent Office Office européen des brevets**

(11) **EP 4 304 631 B1**

(12) **EUROPEAN PATENT SPECIFICATION**

(45) Date of publication and mention of the grant of the patent:
**30.10.2024 Bulletin 2024/44**

(51) International Patent Classification (IPC):
**A61K 38/44** (2006.01)    **A61P 17/02** (2006.01)
**A61P 17/00** (2006.01)    **A61P 27/02** (2006.01)

(21) Application number: **22711023.6**

(22) Date of filing: **07.03.2022**

(52) Cooperative Patent Classification (CPC):
**A61K 38/44; A61P 17/00; A61P 17/02; A61P 27/02**

(86) International application number:
**PCT/EP2022/055727**

(87) International publication number:
**WO 2022/189344 (15.09.2022 Gazette 2022/37)**

(54) **TREATMENT OF EYE DISEASES WITH FRUCTOSYL-AMINO ACID OXIDASE**

BEHANDLUNG VON AUGENERKRANKUNGEN MIT FRUCTOSYLAMINOSÄUREOXIDASE

TRAITEMENT DE MALADIES OCULAIRES AVEC LA FRUCTOSYL-AMINOACIDE OXYDASE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.03.2021 EP 21161313**

(43) Date of publication of application:
**17.01.2024 Bulletin 2024/03**

(73) Proprietor: **Universiteit Gent**
**9000 Gent (BE)**

(72) Inventors:
• **DELANGHE, Joris**
 **9300 Aalst (BE)**
• **VAN AKEN, Elisabeth**
 **9070 Heusden (BE)**

(56) References cited:
**WO-A1-2019/149648**    **WO-A1-2020/053188**
**US-A1- 2005 014 935**

• **EDOARDO CAPUANO ET AL: "Studies on the Effect of Amadoriase from Aspergillus fumigatus on Peptide and Protein Glycation In Vitro", JOURNAL OF AGRICULTURAL AND FOOD CHEMISTRY, vol. 55, no. 10, 1 May 2007 (2007-05-01), pages 4189 - 4195, XP055154303, ISSN: 0021-8561, DOI: 10.1021/jf0700024**
• **SHEN ET AL: "B44: Development of Point-of-Care Hemoglobin A1c Assay based on Enzyme Method", 2013 AACC ANNUAL MEETING, 1 July 2013 (2013-07-01), pages 1 - 296, XP055829755**

Remarks:
The complete document including Reference Table(s) and the Sequence Listing(s) can be downloaded from the EPO website

**Description**

Field of the invention

The present disclosure relates to the application of the enzyme fructosyl-amino acid oxidase (FAOD) -without the addition of proteases- in medical conditions which are characterized by the presence of advanced glycation end products (AGEs).

More specifically, the present disclosure relates to the treatment of eye diseases in humans or animals. AGEs accumulate in the aging eye causing presbyopia, cataract, dry eyes, age-related macular degeneration, glaucoma and diabetic retinopathy.

Hence, the disclosure relates to the in vivo application of FAOD in order to deglycate and inactivate said AGEs. The invention is defined by the appended claims.

Background of the invention

Protein glycation is a process of ageing, where metabolically important sugars react with primary amine groups, forming adducts that can rearrange and react further, eventually leading to cross-links between proteins (Maillard reaction) (1). This ageing process of protein glycation specifically occurs in organs with long-lived proteins such as the eye and the skin.

Lens crystallins, retinal cells, corneal cells, the trabecular meshwork and optic nerve in the eye do not renew during life and are prone to glycation. As a result, the ageing lens gets stiff, causing ageing persons to wear reading glasses (presbyopia). Further on, the lens gets even cloudy, and blindness due to cataract occurs for which no other treatment is available than surgery (2). An increasing amount of AGEs in tear film alters corneal biomechanics and can induce dry eye syndrome (3). With age, AGEs also accumulate in and under the retina, causing age-related macular degeneration (AMD) and blindness in 25% of persons over 75 years of age (2). In addition, the ageing collagenous matrix of the trabecular meshwork and lamina cribrosa of the optic nerve causes blindness due to glaucoma, a disease characterized by high intraocular pressure (4,5). In diabetic patients, age and hyperglycemia are the most prominent risk factors for developing blindness due to diabetic retinopathy (5,6).

The nonsurgical treatment of AGEs-dependent ocular diseases by the enzyme fructosamine-3-kinase (F3K or FN3K) has been described: WO2019149648 discloses a method to treat cataract by administering recombinant F3K and its cofactor(s) to the eye in order to deglycate lens crystallins, and, WO2020053188 discloses a method to treat advanced glycation end product dependent ocular diseases by administering recombinant F3K and its cofactor(s) to the eye .

Fructosyl-amino acid oxidase (FAOD; fructosyl-$\alpha$-L-amino acid: oxygen oxidoreductase (defructosylating), is an enzyme found in many bacteria and yeasts (7,8). FAOD catalyzes the oxidation of the C-N bond linking the C1 of the fructosyl moiety and the nitrogen of the amino group of fructosyl amino acids. Flavin adenine dinucleotide (FAD) acts as its cofactor. It is active on both of fructosyl lysine and fructosyl valine. FAOD-based detection methods for glycated proteins -as for example described by Shen et al. (Abstract B44 of 2013 AACC Annual Meeting) (9) and in US2005/0014935 have been commercially available since 1999. However FAOD is unable to react with most intact glycated proteins, and samples thus require an initial proteolytic digestion step to liberate glycated amino acids or glycated dipeptides. Capuano et al. (J. Agric. Food Chem 2007:4189) show a deglycating effect of FAOD on some low molecular weight proteins such as insulin and conclude that FAOD could be used as a tool to inhibit protein glycation in food systems (10). However an in vivo therapeutic use of FAOD in AGEs-induced conditions in human or animal has never been considered

Brief description of figures

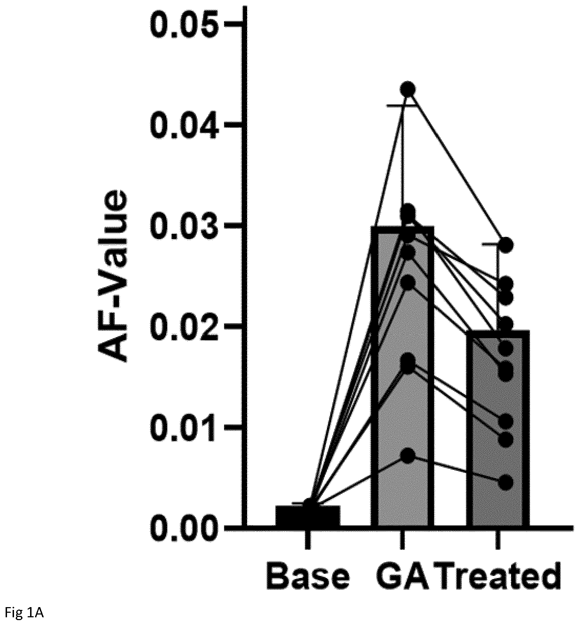

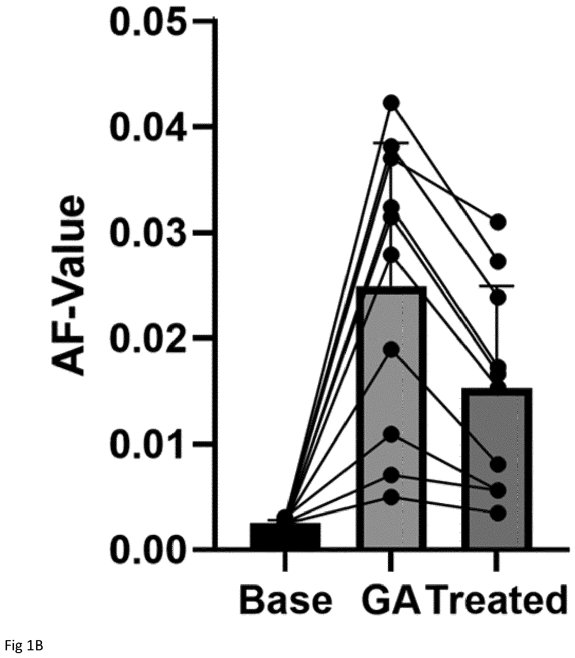

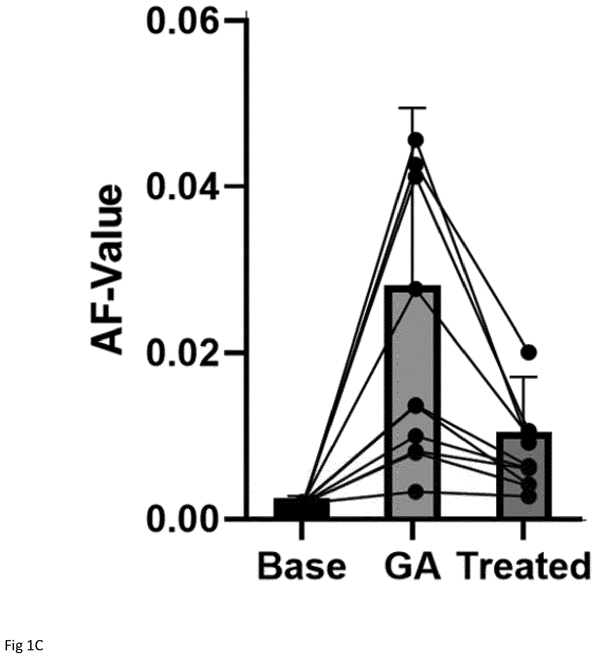

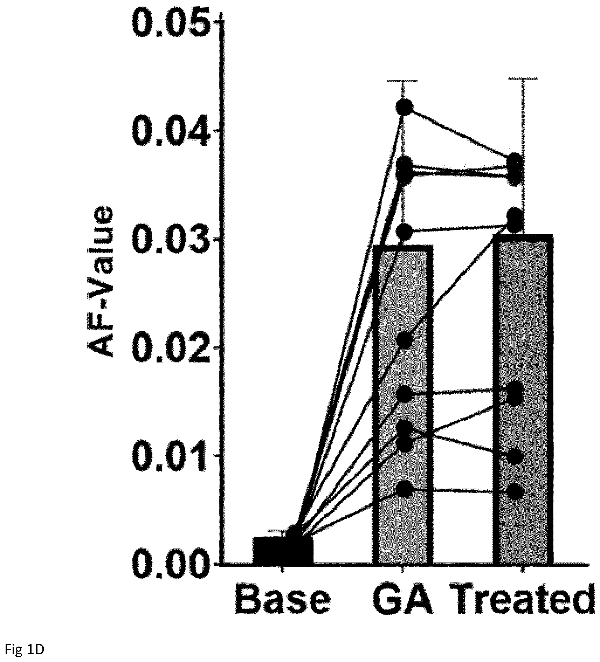

**Figure 1. Fluorescence spectroscopic measurements of porcine retina treated with FAOD alone or in combination with FN3K.**
Mean autofluorescence values and standard deviation of 3 measurements of emission spectra (400 - 600 nm) of porcine retinas (n=40) glycated with glycolaldehyde (GA) and then treated with FN3K + ATP + MgCl$_2$ (fig 1A), FAOD (Fig 1B), combination of FN3K + ATP + MgCl$_2$ and FAOD (fig 1C). For negative control, PBS + ATP + MgCl$_2$ was used (fig 1D). Baseline autofluorescence spectroscopic measurements were performed for all retinas before glycation (base, black first bar), after 3 hr glycation with 25 mmol/L glycolaldehyde (GA, light grey, second bar), and after 3 hr treatment (treated, dark grey, third bar).

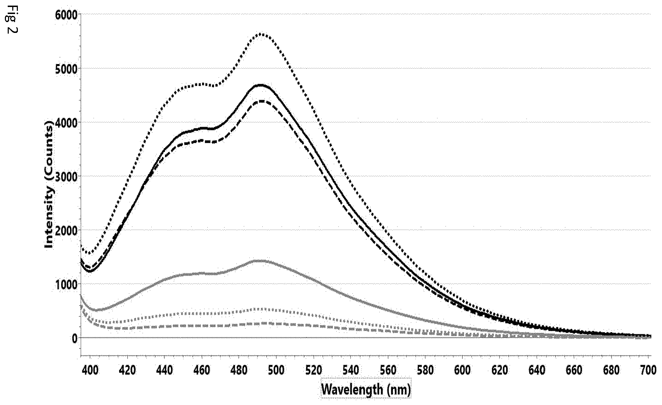

**Figure 2. Fluorescence spectroscopic measurements of human lens fragments treated with FAOD alone or in combination with FN3K.**
Mean autofluorescence values and standard deviation of 3 measurements of emission spectra (400 - 600 nm) of three similar pools of human cataractous lens fragments (n=30) before treatment (black lines) and after treatment (grey lines). The pools were either treated with FAOD (grey dotted line), FN3K + ATP + MgCl$_2$ (grey solid line), or the combination of both FAOD and FN3K + ATP + MgCl$_2$ (grey dashed line).

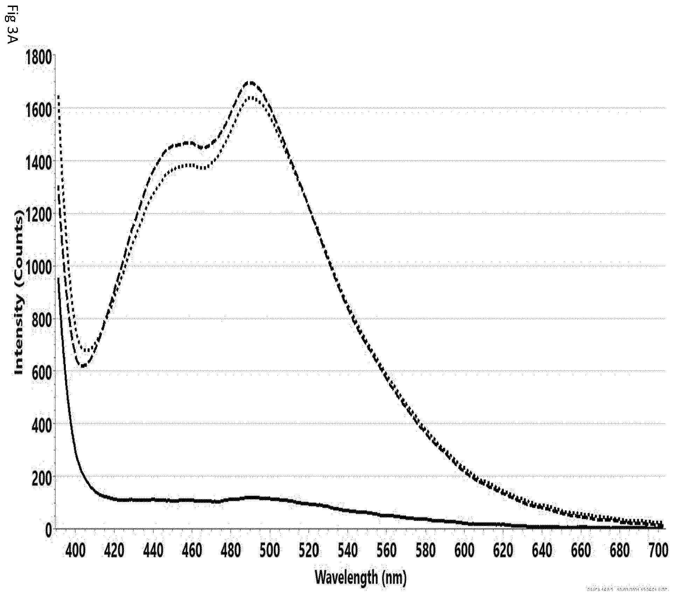

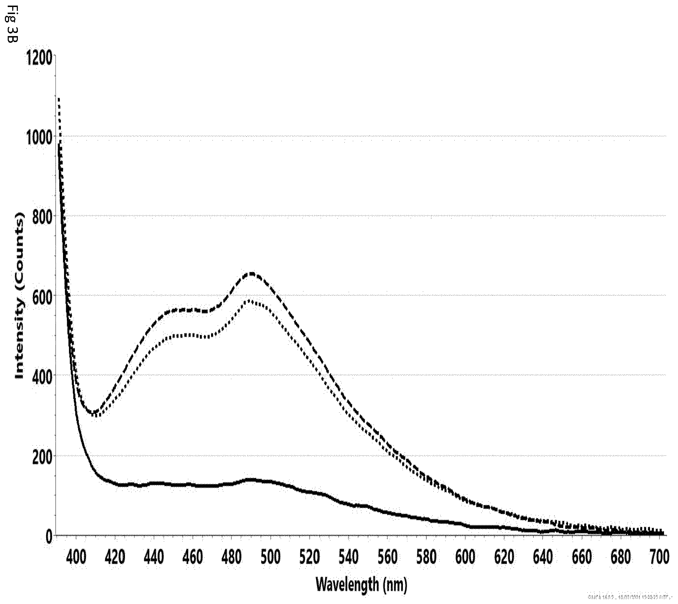

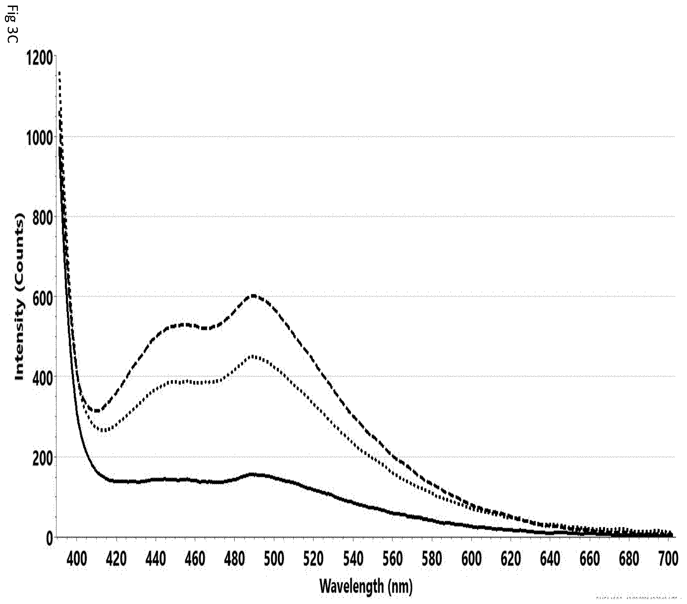

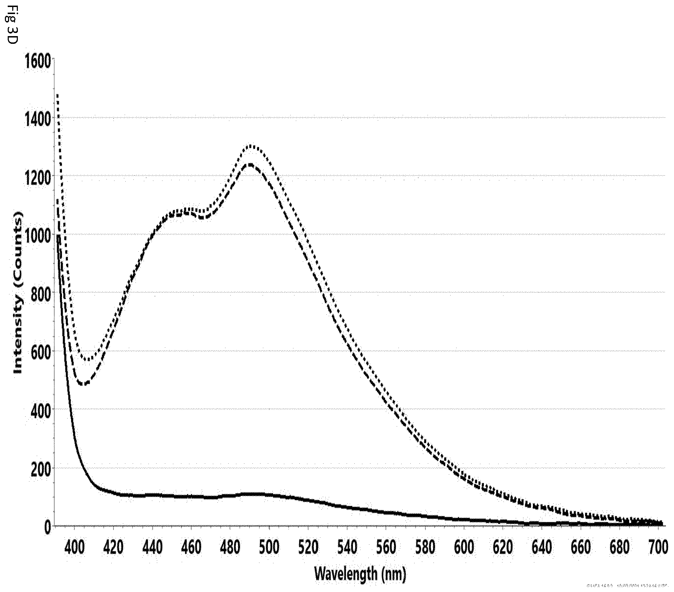

**Figure 3. Fluorescence spectroscopic measurements of human cornea treated with FAOD alone or in combination with FN3K.**
Mean autofluorescence values and standard deviation of 3 measurements of emission spectra (400 - 600 nm) of human cornea fragments glycated with glycolaldehyde (GA) and then treated with FN3K + ATP + MgCl$_2$ (fig 3A), FAOD (Fig 3B), or combination of FN3K + ATP + MgCl$_2$ and FAOD (fig 3C). For negative control, PBS + ATP + MgCl$_2$ was used (fig 3D). Baseline autofluorescence spectroscopic measurements were performed for all cornea fragments before glycation (black curve), after 3 hr glycation with 25 mmol/L GA (dashed curve), and after 3 hr treatment (dotted curve).

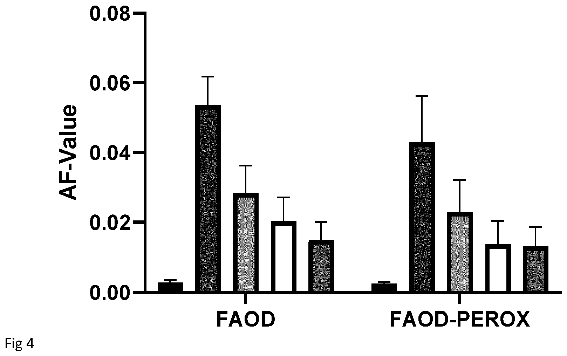

**Figure 4. Fluorescence spectroscopic measurements of porcine retina treated with FAOD and peroxidase.**
Mean autofluorescence values and standard deviation of 3 measurements of emission spectra (400 - 600 nm) of porcine retinas (n=40) before glycation (small black bars), and after 3 hr glycation with 25 mmol/L glycolaldehyde (large dark grey bars). Porcine retinas were then either treated with FAOD alone or in combination with 473 U/mL peroxidase for 1 hr (light grey bars), 2 hr (white bars) or for 3 hr (grey bars).

**Figure 5. Shift of fluorometry of cataractous lens fragments treated with FAOD or with FN3K.** Human lens fragments derived from a pool of senile and diabetic patients were treated for 3 hours with different deglycating enzymes. Mean autofluorescence values of 3 experiments of emission spectra (400 - 600 nm) of three similar pools of human cataractous lens fragments (n=30) before treatment (dashed lines) and after treatment (full lines). The pools were either treated with FN3K + ATP + MgCl$_2$ (fig 5A), or with FAOD +FAD (fig 5B).

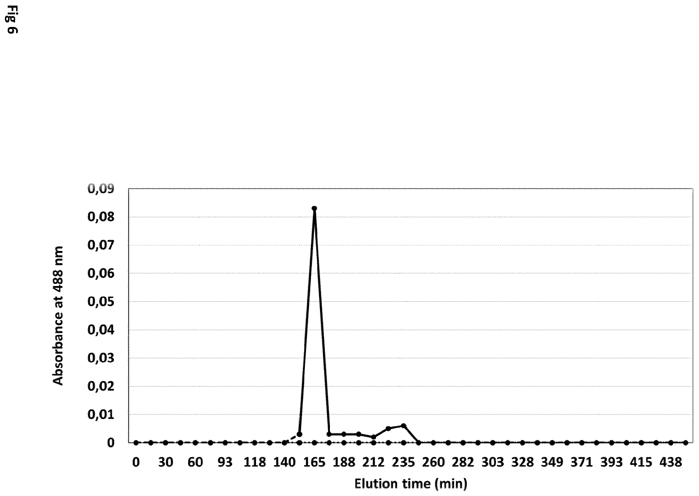

**Figure 6. Gel filtration patterns (Sephadex® G-25 Fine resin) of urea-soluble lens fractions before and after FAOD treatment.** The absorbance at 488 nm (Seliwanoff reaction) of high-molecular mass fructose containing compounds (AGEs) as a function of the elution time are presented. V0 (>5 kDa) indicates the void volume, Vt (<500 Da) the terminal volume. Sharp peak after 165 min and smaller peak after 235 min elution time represent the presence of fructose containing AGEs in lenses before FAOD treatment. Dots on abcis show the total loss of fructose containing AGEs at every elution time point.

**Figure 7. Gain of human lens power after FAOD treatment.**
Lens power of human lenses in (D, water) as a function of the treatment time presented (hours) with saline (Fig 7A). Gain of lens power (D, water) as a function of the treatment time with FAOD (Fig 7B)

**Figure 8. Near-infrared microspectroscopy on stained tissue sections of human retina with AGEs unravels different biochemical changes after FN3K treatment compared to FAOD treatment.** Hotelling's plot of spectral data, analyzed by principal component analysis and partial least squares-discriminant analysis, showing a clear distinction between FN3K-treated Drüsen (squares) and FAOD-treated Drüsen (dots).

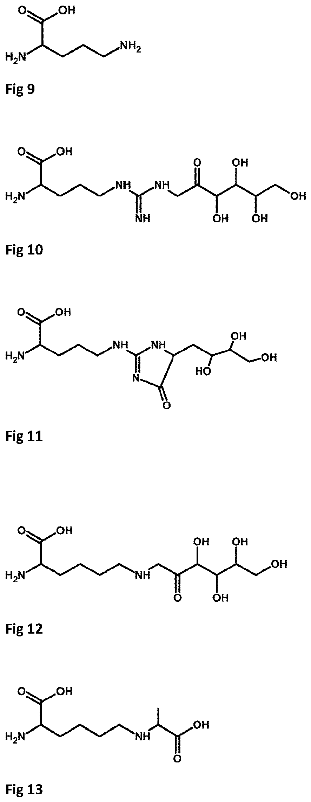

**Figure 9.** Proposed structure for the compound with m/z 133,09695 at RT 0.82 (Compound # A1), detected in arginine-containing samples.

**Figure 10.** Proposed structure for the compound with m/z 337,17098 at RT 0.88 (Compound # A3), detected in arginine-containing samples.

**Figure 11.** Proposed structure for the compound with m/z 319,16064 at RT 0.88 (Compound # A4), detected in arginine-containing samples

**Figure 12.** Proposed structure for the compound with m/z 309,16554 at RT 0.85 (Compound # L1), detected in lysine-containing samples.

**Figure 13** Proposed structure for the compound with m/z 219,13387 at RT 0.89 (Compound # L2), detected in lysine-containing samples.

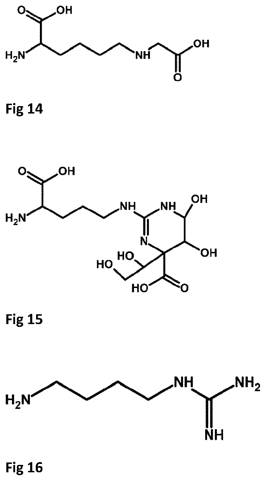

**Figure 14.** Proposed structure for the compound with m/z 205,11825 at RT 0.91 (Compound # L3), detected in lysine-containing samples.

**Figure 15.** Proposed structure for the compound with m/z 351,15047 at RT 0.95 (Compound # A9), detected in arginine-containing samples.

**Figure 16.** Proposed structure for the compound with m/z 131,12904 at RT 0.83 (Compound # A2), detected in arginine-containing samples.

## Summary of invention

The present invention relates in first instance to a composition comprising a fructosyl amino oxidase for use to treat presbyopia, cataract, dry eye syndrome, age-related macular degeneration, diabetic retinopathy and/or glaucoma

The present invention further relates to a composition for use as described above further comprising flavin adenine dinucleotide (FAD).

The present invention also relates to a composition for use as described above further comprising a fructosamine-3-kinase and adenosine tri phosphate (ATP).

The present invention also relates to a composition for use as described above which further comprises magnesium ions.

The present invention further relates to a composition for use as described above which further comprises a peroxidase.

The present invention further relates to a composition as described above wherein said composition is administered as drops or as any other external application, or internal application.

## Detailed description of the invention

The present invention relates to the surprising finding that FAOD -which is shown not to be able to revert protein glycation without the preceding usage of proteinases- is capable to deglycate proteins within eye tissues in vivo and has -as such- a therapeutic effect. Furthermore, the present invention further discloses that FAOD deglycates different proteins than F3K so that using both enzymes simultaneously on the same tissues has an improved (additive) therapeutic effect.

The present invention relates in first instance to a composition comprising a fructosyl amino oxidase for use to treat specific diseases of the eye such as glaucoma, age-related macular degeneration of the eye, diabetic retinopathy, dry eye syndrome, presbyopia and cataract, and AGEs-related appearances of the skin.

In one embodiment, the present invention relates to the surprising findings that the application of fructosyl-amino acid oxidase alone or in combination with fructosamine 3 kinase and its cofactor(s), results in less fluorescence and low intraocular pressure in eye tissues. In other words, the latter treatments lower intraocular pressure and improve vision in patients with AGEs-induced diseases such as glaucoma.

In another embodiment, the present invention relates to the surprising finding that the administration of fructosyl-amino acid oxidase alone or in combination with fructosamine 3 kinase and its cofactor(s), results in less AGEs-induced fluorescence in the retina. In other words, the latter administration restores light transmission and thus vision in patients with AGEs-induced ocular retinopathy such as age-related macular degeneration and diabetic retinopathy.

In another embodiment, the present invention relates to the surprising finding that the administration of fructosyl-amino acid oxidase alone or in combation with fructosamine 3 kinase and its cofactor(s), results in less AGEs-induced fluorescence in the cornea. In other words, the latter administration restores light transmission and thus vision in patients with AGEs-induced ocular keratopathy such as dry eye syndrome.

In yet another embodiment, the present invention relates to the surprising finding that treatment with fructosyl-amino acid oxidase alone or in combination with fructosamine 3 kinase and its cofactor(s), reduces AGEs-induced fluorescence in the lens and thus restores light transmission and vision in patients with AGEs-induced ocular lens diseases such as presbyopia and cataract.

Several embodiments of the present invention thus relate to a composition comprising a fructosyl-amino acid oxidase alone or in combination with a fructosamine 3 kinase and its cofactor(s), for use to treat AGEs-induced ocular diseases such as age-related macular degeneration, diabetic retinopathy, presbyopia, cataract, dry eye syndrome, glaucoma in a human or an animal.

With the term 'fluorescence in eye tissues' is meant the UV fluorescence signal observed after illuminating the eye tissue with UV light. AGEs are quantified based on Maillard-type autofluorescence measurements (excitation wavelength of UV light 365 nm, emission wavelength 390-700 nm).

With the term 'low intraocular pressure' is meant the pressure in the eye measured by any means of tonometer (contact tonometer or non-contact tonometer).

With the term 'eye tissues' is meant all eye tissues wherein AGEs accumulate, such as the lens, the retina, the cornea, the trabecular meshwork, the vitreous, the optic nerve.

With the term 'improve vision' is meant that vision is better in clinical tests after application of fructosyl-amino acid oxidase alone or in combination with fructosamine 3 kinase and its cofactor(s),or in combination with peroxidase to the eye.

With the term 'AGEs-induced diseases' is -with regard to the embodiments of the present invention related to vision- meant all vision threatening diseases in which AGEs are involved, such as presbyopia, cataract, dry eye syndrome, age-related macular degeneration, diabetic retinopathy or glaucoma.

The present invention thus relates to a composition comprising a fructosyl-amino acid oxidase. The term 'a fructosyl-amino acid oxidase (FAOD; fructosyl-$\alpha$-L-amino acid: oxygen oxidoreductase (defructosylating)' relates to any enzyme classified as catalyzing the oxidation of the C-N bond linking the C 1 of the fructosyl moiety and the nitrogen of the amino group of fructosyl amino acids. The reaction proceeds to an unstable Schiff base intermediate, which hydrolyzes to produce glucosone and an amino acid. The enzyme's reduced flavin adenine dinucleotide (FAD) cofactor is then reoxidized by molecular oxygen with the release of hydrogen peroxide.

More specifically, the terms'a fructosyl-amino acid oxidase (FAOD; fructosyl-$\alpha$-L-amino acid: oxygen oxidoreductase (defructosylating)' relates to the enzyme encoded by the gene encoding the fructosyl-amino acid oxidase (fructosyl-a-L-amino acid: oxygen oxidoreductase(defructosylating); EC 1.5.3) of Corynebacterium sp. 2-4-1 which was cloned and expressed in Escherichia coli as described by Sakaue et al. (11). The latter enzyme -as a non-limiting example of an enzyme which can be used in the present invention- can be purchased from-for example- Creative Enzymes, Shirley, NY or can be made using well-known recombinant methods as is for example described by Sakaue et al. (11)

The term 'a fructosamine-3-kinase' relates to enzymes classified as enzymes 2.7.1.171 in -for example-the Brenda enzyme database (www.brenda-enzymes.org). The latter enzymes are part of an ATP-dependent system for removing carbohydrates from non-enzymatically glycated proteins and catalyze the following reaction: ATP + [protein] -N6-D-fructosyl-L-lysine = ADP + [protein]-N6-(3-0-phospho-D-fructosyl)-L-lysine. More specifically, the term 'a fructosamine-3-kinase' relates to -as a non-limiting example- to the human fructosamine-3-kinase having accession number or the National Center for Biotechnology Information (NCBI) Reference sequence number :NP_071441.1 (see https://www.ncbi.nlm.nih.gov/protein/NP 071441). BothWO2019149648 and WO2020053188 describe -for example- the recombinant production of F3K in Pichia pastoris.

It should be further clear that the term 'a fructosamine-3-kinase' and 'a fructosyl amino acid oxidase' relates to the enzymes as described above, but also to functional fragments and variants thereof. The term "functional fragments and variants" relates to fragments and variants of the naturally occurring enzymes. Indeed, for many applications of enzymes, part of the protein may be sufficient to achieve an enzymatic effect. The same applies for variants (i.e. proteins in which one or more amino acids have been substituted with other amino acids, but which retain functionality or even show improved functionality), in particular for variants of the enzymes optimized for enzymatic activity (as is also described further with regard to recombinant enzymes). The term 'fragment' thus refers to an enzyme containing fewer amino acids than the 309 amino acid sequence of the human fructosamine-3-kinase having NCBI Reference sequence number :NP_071441.1 or the 372 amino acid sequence of the fructosyl amino acid oxidase as disclosed by Sakaue et al. (11) and that retains said enzyme activity. Such fragment can -for example- be a protein with a deletion of 10% or less of the total number of amino acids at the C- and/or N-terminus. The term "variant" thus refers to a protein having at least 50 % sequence identity, preferably having at least 51-70 % sequence identity, more preferably having at least 71-90% sequence identity or most preferably having at least 91, 92, 93, 94, 95, 96, 97, 98 or 99 % sequence identity with the 309 amino acid sequence of the human fructosamine-3-kinase having NCBI Reference sequence number :NP_071441.1 or with or the 372 amino acid sequence of the fructosyl amino acid oxidase as disclosed by Sakaue et al. (11) and that retains said enzyme activity.

Hence, orthologues, or genes in other genera and species (than the human fructosamine-3-kinase having NCBI Reference sequence number :NP_071441.1 or than the fructosyl amino acid oxidase as disclosed by Sakaue et al. (11) with at least 50 % identity at amino acid level, and having said enzyme activity are part of the present disclosure. The percentage of amino acid sequence identity is determined by alignment of the two sequences and identification of the number of positions with identical amino acids divided by the number of amino acids in the shorter of the sequences x 100. The latter 'variant' may also differ from the protein having NCBI Reference sequence number :NP_071441.1 or the protein as disclosed by Sakaue et al. (11) only in conservative substitutions and/or modifications, such that the ability of the protein to have enzymatic activity is retained. A "conservative substitution" is one in which an amino acid is substituted for another amino acid that has similar properties, such that one skilled in the art of protein chemistry would expect the nature of the protein to be substantially unchanged. In general, the following groups of amino acids represent conservative changes: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

Variants may also (or alternatively) be proteins as described herein modified by, for example, the deletion or addition of amino acids that have minimal influence on the enzymes activity as defined above, secondary structure and hydropathic nature of the enzyme.

The terms 'adenosine tri phosphate' (ATP), flavin adenine dinucleotide (FAD) and 'magnesium ions' relate to well-known cofactors of the latter enzymes.

The present invention further relates to a composition for use as described above which further comprises a peroxidase. The term 'peroxidase' relates to any well-known enzyme having EC number 1.11.1.x and are capable to break up peroxides, more specifically which are capable to break up hydrogen peroxides which are released during the oxidation of the reduced FAD which is the cofactor of FAOD.

The term 'animal' may relate to any animal such as mammals (dogs, cats, horses, ...), birds and reptiles.

The present disclosure thus relates -in other words- to a method to treat (or prevent) age-related presbyopia, cataract, dry eye syndrome, age-related macular degeneration, diabetic retinopathy, glaucoma in a subject in need thereof wherein said method comprises administering drops of a therapeutically effective amount of a compound comprising a fructosyl amino acid oxidase alone or in combination with a fructosamine-3-kinase and adenosine tri phosphate, and -in certain embodiments of the present invention- magnesium ions and/or peroxidase to the eye of said subject. Methods of treatment are not covered by the claimed invention.

The term 'a therapeutically effective amount' relates to an amount taken from a therapeutic dose ranging between 1 U/mL and 100 U/mL fructosyl amino acid oxidase alone, or in combination with peroxidase between 10 and 946 U/mL, or in combination with fructosamine-3-kinase between 4,17 and 12.5 mg/ml, ATP between 2.50 and 4,17 mM and MgCl$_2$ between 1.00 and 1.67 mM . The latter therapeutic doses can be obtained by mixing 1:1, 1:2, 1:3 or 1:5 a solution of 25 mg/ml fructosamine-3-kinase with a fresh solution of 5mM ATP/2mM MgCl$_2$

It should be clear that besides 'administering drops' said therapeutically effective amounts - which is one way of administration- also other means of administration are envisioned such as -but not limited to- external application such as via gels, and, other internal applications such as suprachoroidal injections or intravitreal injections or subretinal injections or implants everywhere else in or around the eye . Hence, and for example, the present invention therefore relates to a composition comprising a fructosyl amino oxidase alone or in combination with a fructosamine-3- kinase and ATP (and which might further comprise magnesium ions) for use to treat AGEs-induced ocular diseases as age-related macular degeneration, diabetic retinopathy, presbyopia, cataract, dry eye, glaucoma wherein said composition is administered by drops or as any other external application or internal application (such as injections or implants) or any other way of ocular drug delivery (12).

The term 'a therapeutically effective amount' -with regard to F3K- relates to an amount ranging from 10 $\mu$l to 100 $\mu$l taken from a therapeutic dose ranging between about 4,17 and 12.5 $\mu$g/ml fructosamine-3-kinase, 2.50 and 4,17 mM ATP and 1.00 and 1.67 mM MgCl$_2$. The latter therapeutic doses can be obtained by mixing 1:1, 1:2, 1:3 or 1:5 a solution of 25 $\mu$g/ml fructosamine-3-kinase with a fresh solution of 5mM ATP/2mM MgCl$_2$.

The term 'a therapeutically effective amount'-with regard to FAOD- relates to an amount ranging from 10 $\mu$l to 100 $\mu$l taken from a therapeutic dose ranging between 1 U/mL and 100 U/mL fructosyl-amino acid oxidase. FAD concentrations of 2 mmol FAD/mol FAOD can be used for optimal functioning of the enzyme. The solution buffer should have a pH values between 6.8 and 7.7.

The present invention further relates to a composition as indicated above wherein said fructosamine-3-kinase and said fructosyl amino acid oxidase is a recombinant enzyme. The term 'recombinant' refers to fructosamine-3-kinase or fructosyl amino acid oxidase obtained as an outcome of the expression of recombinant DNA encoding for a fructosamine-3-kinase or fructosyl amino acid oxidase inside living cells such as bacteria or yeast cells. Practitioners are further directed to Sambrook et al. Molecular Cloning: A laboratory Manual, 4th ed. , Cold Spring Harbor press, Plainsview, New York (2012) and Ausubel et al. Current Protocols in Molecular Biology (supplement 114), John Wiley & Sons, New York (2016).

More specifically the present invention relates to a recombinant fructosamine-3-kinase which is obtainable by recombinant production in *Pichia pastoris* and, even more specifically, wherein said recombinant fructosamine-3-kinase obtainable by recombinant production in *Pichia pastoris* has the amino acid sequence as given by SEQ ID N° 1 or SEQ ID N°2. SEQ ID N°1 is a construct with an N-terminal cleavable HIS-tag and a caspase 3-cleavable Asp-Glu-Val-Asp (DEVD) linker between the His6 tag and the protein coding sequence which allows for clean removal of the tag. SEQ ID N° 2 is the cleaved version of SEQ ID N° 1.

The amino acid sequences of SEQ ID N°1 and SEQ ID N°2 (and their encoding nucleic acid sequences SEQ ID N3 and SEQ ID N° 4, respective) are as follows:

**SEQ ID N° 1:**
Type: amino acid 1-letter (underlined: His6-tag, *italics: linker,* **bold underlined: caspase cleavage site)**

MHHHHHH*VNGPGS***DEVD*EQLLRAELRTATLRAFGGPGAGCISEGRAYDTDAGPVFVKVNRRT

QARQMFEGEVASLEALRSTGLVRVPRPMKVIDLPGGGAAFVMEHLKMKSLSSQASKLGEQMA

DLHLYNQKLREKLKEEENTVGRRGEGAEPQYVDKFGFHTVTCCGFIPQVNEWQDDWPTFFAR

HRLQAQLDLIEKDYADREARELWSRLQVKIPDLFCGLEIVPALLHGDLWSGNVAEDDVGPII

YDPASFYGHSEFELAIALMFGGFPRSFFTAYHRKIPKAPGFDQRLLLYQLFNYLNHWNHFGR

EYRSPSLGTMRRLLK*

**SEQ ID N°3:**
Type: DNA (underlined: His6-tag, *italics: linker,* bold underlined: caspase cleavage site)

ATGCATCATCATCATCATCAT*GTTAACGGTCCAGGTTCT***GATGAAGTTGAT**GAACAGTTGTT

GAGAGCTGAGTTGAGAACTGCTACTTTGAGAGCTTTTGGTGGTCCAGGTGCTGGTTGTATTT

CTGAGGGTAGAGCTTACGATACTGACGCTGGTCCAGTTTTCGTTAAGGTTAACAGAAGAACT

CAGGCTAGACAGATGTTCGAGGGTGAAGTTGCTTCTTTGGAGGCTTTGAGATCCACTGGTTT

GGTTAGAGTTCCAAGACCAATGAAGGTTATCGACTTGCCAGGTGGTGGTGCTGCTTTTGTTA

TGGAACACTTGAAGATGAAGTCCTTGTCCTCCCAGGCTTCTAAGTTGGGTGAACAAATGGCT

GACTTGCACTTGTACAACCAGAAGTTGAGAGAAAAGTTGAAAGAGGAAGAGAACACTGTTGG

TAGAAGAGGTGAAGGTGCTGAGCCACAATACGTTGACAAGTTCGGTTTCCACACTGTTACTT

GTTGTGGTTTCATCCCACAGGTTAACGAGTGGCAAGATGACTGGCCAACTTTCTTCGCTAGA

CACAGATTGCAAGCTCAGTTGGACTTGATCGAGAAGGACTACGCTGACAGAGAAGCTAGAGA

ATTGTGGTCCAGATTGCAGGTTAAGATCCCAGACTTGTTCTGTGGTTTGGAGATCGTTCCAG

CTTTGTTGCACGGTGATTTGTGGTCTGGTAACGTTGCTGAAGATGACGTTGGTCCAATTATC

TACGACCCAGCTTCTTTCTACGGTCACTCTGAATTCGAGTTGGCTATCGCTTTGATGTTCGG

TGGTTTCCCAAGATCCTTCTTCACTGCTTACCACAGAAAGATCCCAAAGGCTCCAGGTTTCG

ACCAGAGATTGTTGTTGTACCAGTTGTTCAACTACTTGAACCATTGGAACCACTTCGGTAGA

GAGTACAGATCTCCATCCTTGGGTACTATGAAGAGATTGTTGAAGTAA

**SEQ ID N° 2 (= FN3K after N-terminal HIS-tag removal):**
Type: amino acid 1-letter

EQLLRAELRTATLRAFGGPGAGCISEGRAYDTDAGPVFVKVNRRTQARQMFEGEVASLEALR

STGLVRVPRPMKVIDLPGGGAAFVMEHLKMKSLSSQASKLGEQMADLHLYNQKLREKLKEEE

NTVGRRGEGAEPQYVDKFGFHTVTCCGFIPQVNEWQDDWPTFFARHRLQAQLDLIEKDYADR

EARELWSRLQVKIPDLFCGLEIVPALLHGDLWSGNVAEDDVGPIIYDPASFYGHSEFELAIA

LMFGGFPRSFFTAYHRKIPKAPGFDQRLLLYQLFNYLNHWNHFGREYRSPSLGTMRRLLK*

**SEQ** ID N° 4:

Type: DNA

```
GAACAGTTGTTGAGAGCTGAGTTGAGAACTGCTACTTTGAGAGCTTTTGGTGGTCCAGGTGC
TGGTTGTATTTCTGAGGGTAGAGCTTACGATACTGACGCTGGTCCAGTTTTCGTTAAGGTTA

ACAGAAGAACTCAGGCTAGACAGATGTTCGAGGGTGAAGTTGCTTCTTTGGAGGCTTTGAGA
TCCACTGGTTTGGTTAGAGTTCCAAGACCAATGAAGGTTATCGACTTGCCAGGTGGTGGTGC
TGCTTTTGTTATGGAACACTTGAAGATGAAGTCCTTGTCCTCCCAGGCTTCTAAGTTGGGTG
AACAAATGGCTGACTTGCACTTGTACAACCAGAAGTTGAGAGAAAAGTTGAAAGAGGAAGAG
AACACTGTTGGTAGAAGAGGTGAAGGTGCTGAGCCACAATACGTTGACAAGTTCGGTTTCCA
CACTGTTACTTGTTGTGGTTTCATCCCACAGGTTAACGAGTGGCAAGATGACTGGCCAACTT
TCTTCGCTAGACACAGATTGCAAGCTCAGTTGGACTTGATCGAGAAGGACTACGCTGACAGA
GAAGCTAGAGAATTGTGGTCCAGATTGCAGGTTAAGATCCCAGACTTGTTCTGTGGTTTGGA
GATCGTTCCAGCTTTGTTGCACGGTGATTGTGGTCTGGTAACGTTGCTGAAGATGACGTTG
GTCCAATTATCTACGACCCAGCTTCTTTCTACGGTCACTCTGAATTCGAGTTGGCTATCGCT
TTGATGTTCGGTGGTTTCCCAAGATCCTTCTTCACTGCTTACCACAGAAAGATCCCAAAGGC
TCCAGGTTTCGACCAGAGATTGTTGTTGTACCAGTTGTTCAACTACTTGAACCATTGGAACC
ACTTCGGTAGAGAGTACAGATCTCCATCCTTGGGTACTATGAAGAAGATTGTTGAAGTAA
```

The present invention indeed relates -in addition- to the finding that the recombinant fructosamine-3-kinase obtainable by recombinant production in *Pichia pastoris* and having the amino acid sequence as given by SEQ ID N° 1 and 2 are preferred enzymes for treating said AGEs-related conditions . Indeed, the latter enzymes are preferred as 1) their production in Pichia resulted in higher yields of the enzyme compared with the production in -for example-E. coli, 2) the enzymes had a higher purity when analysed on SDS page, and 3) the presence of endotoxin, which is known to provoke an inflammation following administration, can be avoided.

Examples

**Example 1. Fluorescence spectroscopic measurements of porcine retina treated with FAOD alone or in combination with FN3K.** Porcine retinas (n=40) were obtained from a local abbatoir and stored at 4°C until processing. Neural retinas were isolated through dissection by a trained ophthalmologist within 12 h post-mortem, transferred to a sterile 6-well plate (Thermo scientific, Roskilde, Denmark) and frozen at -20°C. A retinal fragment was cut from each frozen retina and added to a 96-well plate for fluorescence measurements (FluoroNunc PolySorp, Thermo Fisher Scientific, Waltham, MA, USA). Subsequently, fluorescence measurements were performed at baseline for each retinal fragment at a fixed distance and 90° angle. AGE-modification was performed by incubation of the retinal fragments with 200 $\mu$L of 25 mM glycolaldehyde dimer (crystalline form, Sigma-Aldrich) in phosphate buffered saline (PBS) for 3 h at 37°C. After incubation, the active agents were carefully washed away, and retinal fragments were stored overnight (4°C) until termination of the chemical reaction was completed. Finally, in vitro deglycation was initiated using a solution containing FN3K (WO2019149648) 250$\mu$g/mL + ATP 5 mmol/L + MgCl$_2$ 2 mmol/L (n=10), FAOD 10 U/mL (n=10) or combination FN3K 250$\mu$g/mL + ATP 5 mmol/L + MgCl$_2$ 2 mmol/L + FAOD 10 U/mL (n=10).

FAOD was purchased from Creative Enzymes, Shirley, NY. Twenty microliters of the solutions were added to each retinal fragment and incubated for 3 h at 37°C. Fluorescence measurements were performed before the addition of deglycating solutions and repeated after the incubation period. As a control experiment, 10 retinal fragments were processed in a similar way. However, the fragments were control treated with PBS + ATP 5 mmol/L + MgCl$_2$ 2 mmol/L after AGE-modification.

AGEs were quantified based on Maillard-type autofluorescence (AF) measurements (excitation 365 nm, emission 390-700 nm) using a Flame miniature spectrometer (FLAME-S-VIS-NIR-ES, 350-1000 nm, Ocean Optics, Dunedin, FL, USA) equipped with a high-power LED light source (365 nm, Ocean Optics) and a reflection probe (QR400-7-VIS-BX, Ocean Optics). AF-values were calculated by dividing the average light intensity emitted per nm for the 407 nm to 677 nm range by the average light intensity per nm over the 342 nm to 407 nm range.

Mean AF measurements show a steap increase after glycation with GA in all retinal fragments. AF in neural retinas decreased with 34% when treated with the FN3K solution (fig 1A), with 38% when treated with the FAOD solution (fig 1B) and with 62% when treated with a solution containing both FN3K and FAOD (fig 1C), while control-treated retinas stayed stable (AF+ 4%) (fig 1D)

**Example 2. Fluorescence spectroscopic measurements of human lens fragments treated with FAOD alone or in combination with FN3K.** Human cataractous lens fragments (n=30) obtained after phacoemulsification surgery, were washed several times with PBS, pooled and stored at 4°C. UV fluorescence was measured in three pools of lens fragments (fig 2). AGEs were quantified based on Maillard-type autofluorescence (AF) measurements (excitation 365 nm, emission 390-700 nm) using a Flame miniature spectrometer (FLAME-S-VIS-NIR-ES, 350-1000 nm, Ocean Optics, Dunedin, FL, USA) equipped with a high-power LED light source (365 nm, Ocean Optics) and a reflection probe (QR400-7-VIS-BX, Ocean Optics). Pooled lens fragments were then treated for 3 hr at 37°C with FN3K (WO2019149648) 250μg/mL + ATP 5 mmol/L + MgCl$_2$ 2 mmol/L (n=10), FAOD 10U/mL (n=10) or combination FN3K 250μg/mL + ATP 5 mmol/L + MgCl$_2$ 2 mmol/L + FAOD 10U/mL (n=10). FAOD was purchased from Creative Enzymes, Shirley, NY. FN3K treatment resulted in a decrease with 68.85% for UV fluorescence measurements at 450 nm and with 69.71% at 490 nm. FAOD treatment resulted in a decrease with 90% for UV fluorescence measurements at 450 nm and with 90.57% at 490 nm. Treatment with combination of both FN3K and FAOD resulted in a decrease with 93.68% for UV fluorescence measurements at 450 nm and with 93.77% decrease at 490 nm (fig 2).

## Example 3. Fluorescence spectroscopic measurements of human cornea treated with FAOD alone or in combination with FN3K.

Human corneas (n=3) were obtained from cadaver eyes and stored at 4°C until processing. Human corneas were isolated through dissection by a trained ophthalmologist within 12 h post-mortem, transferred to a sterile 6-well plate (Thermo scientific, Roskilde, Denmark) and frozen at -20°C. Corneal fragments were cut from each frozen cornea and added to a 96-well plate for fluorescence measurements (FluoroNunc PolySorp, Thermo Fisher Scientific, Waltham, MA, USA). Subsequently, fluorescence measurements were performed at baseline for each corneal fragment at a fixed distance and 90° angle. AGE-modification was performed by incubation of the corneal fragments with 200 μL of 25 mM glycolaldehyde dimer (GA) (crystalline form, Sigma-Aldrich) in phosphate buffered saline (PBS) for 3 h at 37°C. After incubation, the active agents were carefully washed away, and corneal fragments were stored overnight (4°C) until termination of the chemical reaction was completed. Finally, in vitro deglycation was initiated using a solution containing FN3K (WO2019149648) 250μg/mL + ATP 5 mmol/L + MgCl$_2$ 2 mmol/L, FAOD 10 U/mL or combination FN3K 250μg/mL + ATP 5 mmol/L + MgCl$_2$ 2 mmol/L + FAOD 10 U/mL. FAOD was purchased from Creative Enzymes, Shirley, NY. Twenty microliters of the solutions were added to each corneal fragment and incubated for 3 h at 37°C. Fluorescence measurements were performed before the addition of deglycating solutions and repeated after the incubation period. As a control, corneal fragments were processed in a similar way. However, the fragments were control treated with PBS + ATP 5 mmol/L + MgCl$_2$ 2 mmol/L after AGE-modification.

AGEs were quantified based on Maillard-type autofluorescence (AF) measurements (excitation 365 nm, emission 390-700 nm) using a Flame miniature spectrometer (FLAME-S-VIS-NIR-ES, 350-1000 nm, Ocean Optics, Dunedin, FL, USA) equipped with a high-power LED light source (365 nm, Ocean Optics) and a reflection probe (QR400-7-VIS-BX, Ocean Optics). AF-values were calculated by dividing the average light intensity emitted per nm for the 407 nm to 677 nm range by the average light intensity per nm over the 342 nm to 407 nm range.

Mean AF measurements show a steap increase after glycation with GA in all corneal fragments. AF in corneal fragments decreased with 3% when treated with the FN3K solution (fig 3A), with 12 % when treated with the FAOD solution (fig 3B) and with 27 % when treated with a solution containing both FN3K and FAOD (fig 3C), while control-treated retinas showed slight increase in AF (fig 3D).

**Example 4. Fluorescence spectroscopic measurements of human retina treated with FAOD and peroxidase.** Porcine retinas (n=40) were obtained from a local abbatoir and stored at 4°C until processing. Neural retinas were isolated through dissection by a trained ophthalmologist within 12 h post-mortem, transferred to a sterile 6-well plate (Thermo scientific, Roskilde, Denmark) and frozen at -20°C. A retinal fragment was cut from each frozen retina and added to a 96-well plate for fluorescence measurements (FluoroNunc PolySorp, Thermo Fisher Scientific, Waltham, MA, USA). Subsequently, fluorescence measurements were performed at baseline for each retinal fragment at a fixed distance and 90° angle. AGE-modification was performed by incubation of the retinal fragments with 200 μL of 25 mM glycolaldehyde dimer (GA) (crystalline form, Sigma-Aldrich) in phosphate buffered saline (PBS) for 3 h at 37°C. After incubation, the active agents were carefully washed away, and retinal fragments were stored overnight (4°C) until termination of the chemical reaction was completed. Finally, in vitro deglycation was initiated using a solution containing FAOD 10 U/mL alone or in combination with peroxidase 473 U/mL FAOD was purchased from Creative Enzymes, Shirley, NY. Twenty microliters of the solutions were added to each retinal fragment and incubated for respectively 1 hr, 2 hr or 3 hr at 37°C. Deglycating enzymes were carefully washed away with PBS and AGEs were quantified based on Maillard-type autofluorescence (AF) measurements (excitation 365 nm, emission 390-700 nm) using a Flame miniature spectrometer (FLAME-S-VIS-NIR-ES, 350-1000 nm, Ocean Optics, Dunedin, FL, USA) equipped with a high-power LED light source (365 nm, Ocean Optics) and a reflection probe (QR400-7-VIS-BX, Ocean Optics). AF-values were calculated by dividing the average light intensity emitted per nm for the 407 nm to 677 nm range by the average light intensity per nm over the 342 nm to 407 nm range.

Mean AF measurements show a steap increase after glycation with GA in all retinal fragments (fig 4). AF in neural retinas decreased similarly with 47 % when treated for 1 hr with FAOD solution alone or in combination with peroxidase. After 2 hr incubation with deglycating solutions however, AF in neural retinas decreased with another 40% when treated with FAOD solution in combination with peroxidase as compared to 29% when FAOD solution was used only. After 3 hr incubation, AF in neural retinas decreased further with 5% when treated with FAOD solution in combination with peroxidase as compared to 26% when FAOD solution was used only, suggesting a faster deglycation process when deglycating FAOD solution with peroxidase is used.

**Example 5. Measurement of visual function and intraocular pressure in dogs in vivo after treatment with FAOD in combination with FN3K.** 4 dogs (a chihuahua dog of 11 years old, a poodle dog of 8 years old, a maltese dog of 8 years old and a shepherd dog of 7 years old) with blinding cataract in both eyes were treated with drops containing a combination of FAOD and FN3K for 4 weeks in the right eye and with a mutant inactive FN3K enzyme at the left eye (WO20200053188). 2 out of 4 dogs, the chihuahua dog and the shepherd dog, had developed cataract recently within 3 weeks. The other 2 dogs had an older cataract (longer than 3 months). Treatment consisted of 6 drops with 5 minutes interval at day 1, and then weekly for 4 weeks (4 treatments in total). Drops in the right eye (RE) contained 100 U/mL FAOD and 70 $\mu$g/mL FN3K , 5 mmol/L ATP and 2 mmol/L MgCl2. FAOD was purchased from Creative Enzymes, Shirley, NY. As a control, drops in the left eye (LE) contained 70 $\mu$g/mL mutant FN3K, 5 mmol/L ATP and 2 mmol/L MgCl$_2$. Visual function (VF) was tested in three different visual tests before treatment (baseline) and afterwards weekly during treatment. All tests were performed for RE and LE separately. In the table test the dog scored positive (+) if the dog reached out with his legs when coming closer to a table, in the cotton wad test the dog scored positive (+) if he searched for the cotton wad thrown on a table, in the trail test the dog scored positive (+) if he walked from entrance to exit without bumping into chairs and boxes put randomly on the trail.

Table 1 shows the improvement in visual function in the treated RE of 2 out of 4 dogs (chihuahua dog and shepherd dog) already after 2 weeks of treatment. The dogs that showed fast improvement were the dogs that had developed cataract recently within 3 weeks.

|  | VF baseline | VF 1 week | VF 2 weeks | VF 3 weeks | VF 4 weeks |
|---|---|---|---|---|---|
| Chihuahua RE Chihuahua LE | -/-/- -/-/- | -/-/- -/-/- | **+/+/+** -/-/- | **+/+/+** -/-/- | **+/+/+** -/-/- |
| Shepherd RE Shepherd LE | -/-/- _ | -/-/- -/-/- | **+/+/+** -/-/- | **+/+/+** -/-/- | **+/+/+** -/-/- |

Secondly, effect of treatment with deglycating enzymes on intraocular pressure (IOP) was evaluated in 4 dogs (a chihuahua dog of 11 years old, a poodle dog of 8 years old, a maltese dog of 8 years old and a shepherd dog of 7 years old). Before measuring IOP, an anaesthetic drop was added to the cornea (oxybuprocaine) and IOP was then measured by applanation tonometry with the Tono-pen vet (Reichert, NY, USA). IOP was measured before and 30 minutes after treatment of RE (combination of FAOD and FN3K enzymes) and LE (mutant inactive FN3K enzyme). Treatment consisted of 6 drops with 5 minutes interval. Drops in the right eye (RE) contained 100 U/mL FAOD and 70 $\mu$g/mL FN3K, 5 mmol/L ATP and 2 mmol/L MgCl2. FAOD was purchased from Creative Enzymes, Shirley, NY, USA. As a control, drops in the left eye (LE) contained 70 $\mu$g/mL mutant inactive FN3K (WO20200053188), 5 mmol/L ATP and 2 mmol/L MgCl$_2$. In the RE, high IOP (> 13 mmHg) before treatment could be noted in 2 dogs: in the maltese dog up to 22 mmHg and in the shepherd dog up to 14 mm Hg. After treatment with the combination of deglycating enzymes (combination of FAOD and FN3K) IOP dropped with 54% in the maltese dog and with 29% in the shepherd dog. In the other 2 dogs, IOP of the RE was low (< 13 mmHg) before treatment and stayed low (< 13 mmHg) after treatment with deglycating enzymes. In all 4 dogs, IOP in LE was low (< 13 mmHg) before treatment and stayed low (< 13 mmHg) after treatment with mutant inactive FN3K enzyme.

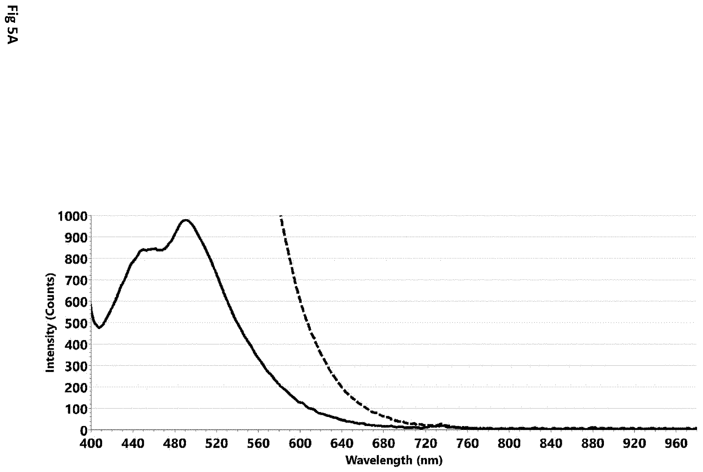

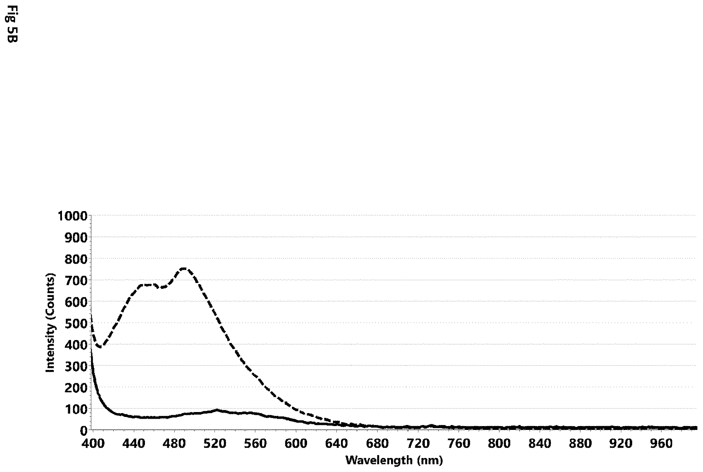

**Example 6 (fig 5) Different fluorescence patterns show different substrate specificity for FN3K and FAOD.** Human cataractous lens fragments (n=30) obtained after phacoemulsification surgery, were washed several times with PBS, pooled and stored at 4°C. As age-related increase is not completely similar for all AGEs in diabetic patients compared to non-diabetic patients, the pool of lens fragments was extended and also senile patients with diabetes were included (13). AGEs were quantified based on Maillard-type autofluorescence (AF) measurements (excitation 365 nm, emission 390-700 nm) using a Flame miniature spectrometer (FLAME-S-VIS-NIR-ES, 350-1000 nm, Ocean Optics, Dunedin, FL, USA) equipped with a high-power LED light source (365 nm, Ocean Optics) and a reflection probe (QR400-7-VIS-BX, Ocean Optics). Two similar pools of lens fragments were then treated for 3 hr at 37°C either with FN3K (WO2019149648) 250μg/mL + ATP 5 mmol/L + MgCl$_2$ 2 mmol/L (n=15), either with FAOD 10U/mL (n=15). FAOD was purchased from Creative Enzymes, Shirley, NY. Following incubation with FN3K treatment, a major decrease is observed in autofluorescence in the spectrum of AGEs (450-500 nm). As a result, two maxima can be observed after FN3K treatment, one large at 500 nm and a second smaller peak of autofluorescence at 450 nm with a major decrease of autofluorescence at 450 nm. In contrast, fluorescence pattern after FAOD treatment is different with complete loss of autofluorescence signal at 450 nm but with a smaller new peak observed at 520 nm representing secondary fluorescent products of the former AGEs (14). Thus, following FAOD treatment but not FN3K treatment, the relative amount of loss of autofluorescence after FAOD treatment is shifted with a new autofluorescence peak at 520 nm.

**Example 7 (fig 6) Different gel filtration pattern of urea-soluble pooled human cataractous lens fragments show different substrate specificity for FN3K and FAOD**

Human lens fragments were obtained following phacoemulsification during cataract surgery from 10 patients. Non-diabetic and diabetic patients were included. After surgery, the conservation fluid containing the lens fragments was centrifuged (1902× g, 5 min, 21 ∘C) and the supernatant was removed. Afterwards, pooled fragments (20 mg) were incubated for 3 h at 37 ∘C in 200 μL of a solution containing FAOD (3.83 U/mL). Untreated pooled fragments (20 mg) were stored under the same conditions. Since it is well known that cataracts are associated with strong increases in water-insoluble proteins, urea-soluble (water-insoluble) proteins from both untreated and treated lens fragments were extracted in 6 M urea in PBS for 4 h at 4 ∘C in Eppendorf SafeLock Tubes (Hamburg, Germany). After centrifugation (16,000× g, 10 min, 21 ∘C), the supernatant was retained for gel filtration. Gel filtration of untreated and FAOD-treated lens fragments was carried out on a chromatography column (length: 60 cm, diameter: 15 mm) packed with Sephadex G-25® Fine resin (Sigma-Aldrich) in order to assess the molecular mass of fructose containing lens compounds such as AGEs. Following fractionation, the presence of AGEs was first checked based on Maillard-type autofluorescence (AF) measurements (excitation 365 nm, emission 390-700 nm) using a Flame miniature spectrometer (FLAME-S-VIS-NIR-ES, 350-1000 nm, Ocean Optics, Dunedin, FL, USA) equipped with a high-power LED light source (365 nm, Ocean Optics) and a reflection probe (QR400-7-VIS-BX, Ocean Optics). Autofluorescence peaks for FAOD treated lens fragments were detected at 520 nm, while autofluorescence peaks for FN3K treated lens fragments which were detected at 450 nm and 500 nm (15). Next, all individual fractions were photometrically tested using the resorcinol-HCl (Seliwanoff) reaction, a well-known color reaction for ketoses (16). In this reaction, a 50 μL sample was added to 100 μL resorcinol (9 mM, Sigma-Aldrich) and 1 mL hydrochloric acid (9 M, Sigma-Aldrich). Following incubation in a boiling water bath for 5 min, the developed color was read photometrically in a standard 10 mm cuvette at 488 nm. Gel filtration of untreated lens fragments showed a sharp peak at 165 min elution time corresponding to a molecular mass of ± 2500 Da and a second minor peak at 235 min elution time corresponding to 1660 Da before treatment, which indicates the presence of high-molecular mass structures such as cross-linked AGEs. In FN3K treated lens fractions, a series of ketoses (fructose-derived AGEs) with molecular mass ranging between 1500 and 2500 Da was detected (15). In contrast, fructose-containing AGEs were completely absent in human cataractous lens fragments after treatment with FAOD, and no peak was detected anymore at any time point of elution. Treatment with FAOD thus not only results in components with different molecular mass, but also with a different structure compared to treatment with FN3K.

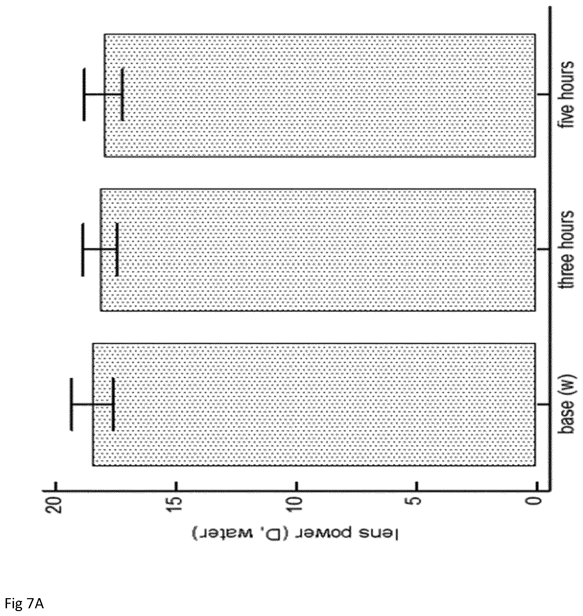

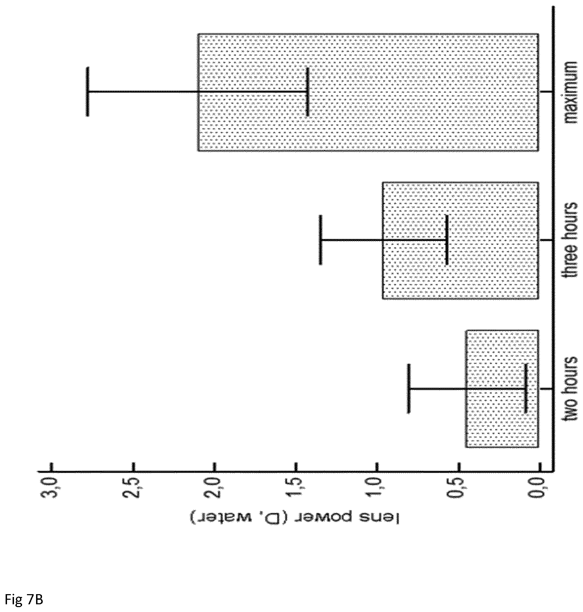

**Example 8 (Figure 7). Gain of human lens power after FAOD treatment**

Human lenses were obtained from cadaver eyes that were rejected for corneal transplantation (Biobank Antwerpen, Antwerpen Belgium, ID71030031000). Eyes were dissected and lenses were removed by a skilled ophthalmic surgeon. Lenses were incubated with FAOD or PBS. Lens power was measured before treatment and every consecutive hour up to 4 hr. The focal distance measurement is based on the thin lens formula $\frac{1}{f} = \frac{1}{S_1} + \frac{1}{S_2}$ that relates the focal distance of the lens $f$ with the object distance from the lens $S_1$ and the image distance from the lens $S_2$. An object will appear in focus when the three quantities fulfill the thin lens formula. On the other hand, the magnification M of an optical system can be expressed as $M = \frac{S_2}{S_1}$. By measuring the total distance between object and image plane $L = S_1 + S_2$ and the magnification M, the focal distance of the lens-under-test can be easily obtained using the equation $L/f = (1 + M)(1 + \frac{1}{M})$. Since the focal distance of the lens-under-test is quite small (about 8 mm), it is impractical to image directly onto a CMOS sensor and an extra imaging lens is used. A high resolution CMOS sensor is used (Basler ace - acA2000-165uc, 2040 x 1086 pixels and pixel size 5.5 $\mu$m) in combination with a fixed focal length imaging lens (25mm/F1.4 59871 Edmund optics, New Jersey). The object consists of a periodic pattern of dark and white lines. First, a reference image is taken without the lens-under-test by placing the camera at the position where the image is in focus. Then, the lens-under-test is placed a random position from the object and the distance of the camera is repositioned such that the image of the object is in focus. By measuring the period of the lines imaged by the camera, the magnification can be calculated and with the position of the camera, finally the focal distance can be calculated. The measurement method was verified by performing the procedure for a known aspheric lens with focal distance 4.03 mm (Thorlabs C340TMD-A) resulting in an error of less than 1% on the focal length. As the quality of the lens-under-test (due to haze and aberrations) is considerably worse than a glass lens, possible issues with focusing the image can be dealt with by repeating the procedure for different distances between object and lens-under-test and averaging the result.

Lens power at baseline (fig 7A) was 17 $\pm$ 0.95 D and did not change when treated with saline. Lens power was measured in air and converted to lens power in water (refractive index of lens in air is 1.5, refractive index of lens in water is 1.13).Lens power increased after 2 hours of FAOD treatment with 0.45 $\pm$ 0.35 D, after 3 hours of FAOD treatment with 0.96 $\pm$ 0.96 D and after 5 hours (maximum) with 2.11 $\pm$ 0.67 (fig 7B). FOAD treatment of human lenses thus results in increased lens power, which reflects a direct relationship with the mechanical properties of the lens (17).

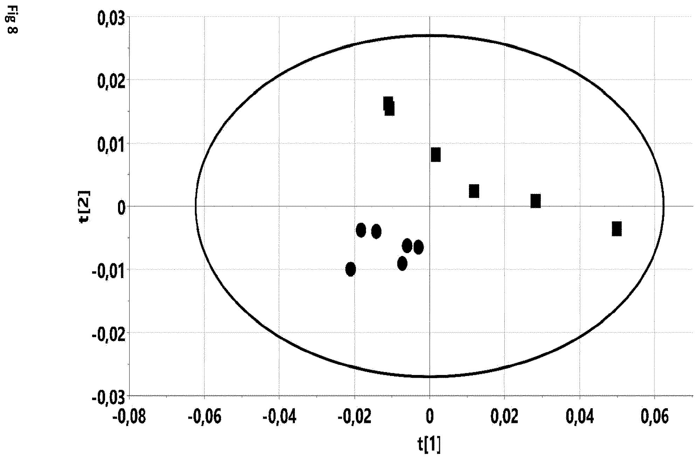

**Example 9 (figure 8) Near-infrared microspectroscopy on stained tissue sections of human retina with AGEs unravels different biochemical changes after FN3K treatment compared to FAOD treatment.**

Donor eyes were obtained from 2 patients with stage 3 AMD (age > 70 years). After tissue sectioning, samples were deparaffinized prior to treatment by consecutive submerging in xylene (3 $\times$ 1.5 min), alcohol (90% 2 $\times$ 1 min; 75% 1 $\times$ 1 min) and rinsing in water. Slides were dried for 10 min at 60 $\circ$C. For control treatment, one section was covered with 1 mL of ATP/MgCl2 solution For FN3K treatment, an adjacent section was treated using 1 mL of FN3K solution (FN3K (WO2019149648) 250$\mu$g/mL + ATP 5 mmol/L + MgCl$_2$ 2 mmol/L. For FAOD treatment, an adjacent section was treated using 1 mL of FAOD solution 3.83U/mL. The sections were incubated at 37 $\circ$C for 24 h. After incubation, tissue sections were carefully washed with distilled water and dried overnight at 37 $\circ$C. Sections were then stained and cover-slipped. Infrared (IR) microspectroscopy combines light microscopy with IR spectroscopy and is a powerful analytical technique to obtain biochemically selective visualizations of tissue sections (18,19)IR spectroscopy is based on the principle that different regions of IR light are absorbed by various molecules within tissues (e.g., carbohydrates, proteins and lipids) (19,20).In a typical IR microspectroscopy system, visible light is employed to visualize and target areas of interest on tissue sections. Once that particular region is found (e.g., a specific drusen), the system switches to the IR configuration and IR light is beamed onto the predefined target (19). To obtain a chemical fingerprint of drusen lesions on the same tissue sections used for light microscopic examination, Fourier transform near-infrared (FT-NIR) transmission microspectra were recorded with a Bruker Hyperion 2000 microscope coupled to a Bruker Vertex 80v FTIR spectrometer (Bruker, MA, USA) operating with a halogen light source, a CaF2 beam splitter and an InGaAs detector. The objective magnification of the microscope was set at 15$\times$ and the aperture at 20 $\mu$m $\times$ 20 $\mu$m. The background was collected with 800 co-adds. Spectra were recorded at a resolution of 16 cm-1 in the range from 12,000 to 4000 cm-1 (800 scans). Spectral data analysis was performed using SIMCA software version 15.0 (MKS Data Analytics Solutions, Malmö, Sweden). Different preprocessing steps were performed to minimize irrelevant light scatter and standardize the spectroscopic signals. Differentiation was performed to accentuate small structural differences and reduce baseline effects (21).Standard normal variate normalization (SNV) was performed to eliminate multiplicative scaling effects and additive baseline offset variations. After preprocessing, spectral data were analyzed by unsupervised pattern recognition methods, such as principal component analysis (PCA), and supervised pattern recognition methods, such as partial least squares-discriminant analysis (PLS-DA),a useful method to illustrate which variables are responsible for discrimination between 2 distinct groups. The resulting Hotelling's plot shows a clear distinction between FN3K-treated Drüsen and FAOD-treated Drüsen.

**Example 10**

Mixtures of fructose and lysine as well as mixture of fructose and arginine, as well as glucose and arginine and glucose-lysine were incubated. After an incubation at 37°C for one week, a portion of the mixture was digested by respectively fructosyl amino oxidase (FAOD, 38.3 U/mL) and fructosamine 3 kinase (F3K, 250 $\mu$g/mL; ATP, 5 mmol/L; MgCl$_2$, 2 mmol/L). An untargeted metabolite profiling approach based on ultra-high performance liquid chromatography (UHPLC) coupled with high resolution mass spectrometry (HRMS) was applied.

**Material and methods**

*Samples*

Mixtures prior to enzyme treatment (group 1):

- Mixture of glucose (100 mg/mL) and arginine (100 mg/mL) in water, incubated at 37°C for one week.

- Mixture of fructose (100 mg/mL) and arginine (100 mg/mL) in water, incubated at 37°C for one week.

- Mixture of glucose (100 mg/mL) and lysine (100 mg/mL) in water, incubated at 37°C for one week.

- Mixture of fructose (100 mg/mL) and lysine (100 mg/mL) in water, incubated at 37°C for one week.

-

- Mixtures after enzyme treatment (group 2):

- Mixture Glu-Arg treated with FAOD,

- Mixture Glu-Arg treated with FN3K,

- Mixture Fru-Arg treated with FAOD,

- Mixture Fru-Arg treated with FN3K,

- Mixture Glu-Lys treated with FAOD,

- Mixture Glu-Lys treated with FN3K, .

- Mixture Fru-Lys treated with FN3K,

In addition, solutions of glucose, fructose, arginine and lysine were also provided for the purpose of optimization and the study of the MS fragmentation pattern of the these compounds.

*UHPLC-HRMS conditions*

The chromatographic separation was achieved on an Accela 1250 pump (Thermo Fisher Scientific), using a Zorbax RRHD Eclipse Plus reverse-phase C18 column (100A, 1.8 $\mu$m, 100 mm $\times$ 2.1 mm). The mobile phase consisted of 0.1 % (v/v) formic acid in water (eluent A), and 0.1 % (v/v) formic acid in methanol (eluent B). A gradient elution programme was applied as follows: 0 - 0.5 min: 5 % B, 0.5 - 20.0 min: 5 - 99 % B, 20.0 - 21.0 min: 99 % B, 21.0 - 24.0 min: 99 - 5 % B, 24.0 - 28.0 min: 5 % B. The mobile phase flow rate was 0.3 mL/min. The column temperature was set at 40°C and temperature of the autosampler was 10°C. The injection volume was 5 $\mu$L.

High-resolution accurate mass and tandem mass spectrometry (MS/MS) fragmentation data were obtained using a Q-Exactive hybrid quadrupole-Orbitrap mass spectrometer (Thermo Fisher Scientific) equipped with a with heated-electrospray ionization (HESI-II) interface. The instrument was operated in the positive ionization mode. Data acquisition included full MS and data dependent MS/MS scans. The ionization source parameters were as follows: a spray voltage of 3.0 kV, a capillary temperature of 350°C, a heater temperature of 375°C, a sheath gas flow rate of 45 arbitrary units (a.u.), an auxiliary gas flow rate of 10 a.u. Daily external calibration of the HRMS was performed using the Calmix solution from Thermo Scientific over a mass range of 138-1721 Da. Online mass calibration using diisooctyl phthalate ($C24H38O4$) as a lock mass was enabled.

Instrument control was carried out by Xcalibur 4.2 software (Thermo Fisher Scientific). For data processing, both Xcalibur and Compound Discoverer 3.3 software (Thermo Fisher Scientific) were used.

**Results**

*Detection of relevant compounds in the investigated samples*

Processing of data generated by the UHPLC-HRMS/MS analysis revealed the presence of a large number of compounds in the investigated samples ($\pm$ 800 distinct compounds were detected in each sample). The majority of compounds that were detected in a given sample from group 2 (i.e. mixture of a specific amino acid and sugar incubated at 37°C, and subsequently treated by an enzyme) were also present in the corresponding sample from group 1 (i.e. mixture of that specific amino acid and sugar incubated at 37°C, but not treated enzymatically). Therefore a detected MS peak was considered to be relevant (i.e. a potential advanced glycation end-product (AGE)) when the following criteria were met:

- The compound is not present in the blank (water).
- For a given sample from group 1, the detected compound is not present in the sample obtained using the same sugar and a different amino acid.
- For a given sample from group 2, the detected compound is not present in the sample obtained using the same sugar and enzyme but a different amino acid.

Forty and 19 relevant compounds (that could possibly be AGEs) were selected for arginine-containing and lysine-containing samples, respectively.

COMPARATIVE ANALYSIS MS FAOD AND F3K digestion

**Disappearing AGEs (A = arginine based, L = lysine -based)**
**STRONG =: more than 80% reduction (vs. untreated sample)**
**WEAK: between 20 and 40% reduction**
**NOT ACTIVE: less than 20% difference**

| # | [M=H] m/z | Molecular formula | annotation | FAOD effect | F3K effect |
|---|---|---|---|---|---|
| A1 | 133.09695 | C5H12N2O2 | ornithine | STRONG | WEAK |
| A3 | 337.17098 | C12H24N4O7 | Fructosyl-arginine/glucosylarginine | STRONG | NOT ACTIVE |
| A4 | 319.16064 | C12H22N4O6 | Imidazolone A | STRONG | NOT ACTIVE |
| | | | | | |
| L1 | 309.16554 | C12H24N2O7 | Fryctosyl-lysine/glucosyl-lysine | STRONG | WEAK |
| L2 | 219.13387 | C9H18N2O4 | carboxyethyllysine | STRONG | WEAK |
| L3 | 205.11825 | C8H16N2O4 | carboxymethyllysine | STRONG | WEAK |

**Formed products**

| # | [M=H] m/z | Molecular formula | annotation | FAOD effect | F3K effect |
|---|---|---|---|---|---|
| A9 | 351.15047 | C12H22N4O8 | Product from arginine, 3-deoxyglycosone and glyoxal | VERY STRONG (> 100 fold increase) | Not detected |
| A2 | 131.12904 | C5H14N4 | agmatine | STRONG (1.8 (glu-Arg) -9.6 fold (Fru-Arg)increase) | Variable (depending on starting mixture) |

14

**CONCLUSION:** Both F3K and FAOD can break down a variety of Advanced glycation end products. FAOD recognize substrates that are not recognized by F3K (compounds #A3 and #A4). The effect of FAOD on AGE degradation is generally more pronounced than that of F3K

References

1. Avery NC et al. The effects of the Maillard reaction on the physical properties and cell interactions of collagen. Pathologic Biologic 2006 54:387-95

2. Nagaraj RH, Linetsky M, Stitt AW. The pathogenic role of Maillard reaction in the eye. Amino Acids. 2012 42:1205-1220.

3. Alves M, Calegari VC, Cunha DA, Saas MJA, Velloso LA, Rocha EM. Increased expression of advanced glycation end-products and their receptor, and activation of nuclear factor kappa-B in lacrimal glands of diabetic rats. Diabetologia 2005 48:2675-2681.

4. Sadowska-Bartosz I, Bartosz G. Effect of glycation inhibitors on aging and age-related diseases. 2016 160:1-18

5. Bejarano E, Taylor A. Too sweet: Problems of protein glycation in the eye. Exp Eye Res. 2019 178:255-262.

6. Cheloni R, Gandolfi SA, Signorelli C, Odone A. Global prevalence of diabetic retinopathy: protocol for a systematic review and meta-analysis. BMJ Open 2019 9:e022188. doi:10.1136/bmjopen-2018-022188

7. Ferri S, Kim S, Tsugawa W, Sode K. Review of fructosyl amino acid oxidase engineering research; a glimpse into the future of hemoglobin A1c biosensing. J Diabetes science and technology 2009 3:585-592.

8. Lin Z, Zheng J. Occurrence, characteristics, and applications of fructosyl amine oxidases (amadoriases). Appl Microbiol Biotechnol 2010 86:1613-1619

9. Shen et al. (Abstract B44 of 2013 AACC Annual Meeting)

10. Capuano et al. (J. Agric. Food Chem 2007:4189)

11. Sakaue R, Hiruma M, Kajiyama N, Koyama Y. Cloning and expression of fructosyl-amino acid oxidase gene grom Corynebacterium sp. 2-4-1 in Escherichia coli. Biosci Biotechnol Biochem 2002 66:1256-61

12. Gote V, Sikder S, Sicotte J, Pal D. Ocular drug delivery: present innovations and future challenges. J Pharmacol Exp Ther 2019 370:602-624

13. Tessier F, Obrenovich M, Monnier VM. Structure and mechanism of formation of human lens fluorophore LM-1. Relationship to vesperlysine A and the advanced Maillard reaction in aging, diabetes and cataractogenesis. J Biol Chem 1999 274:20796-20804

14. Rajan M and Beedu SR. Spectroscopic and biochemical correlations during the course of human lens aging. BMC Ophthalmology 2006 6:10

15. De Bruyne S, Loes van Schie L, Jonas Himpe J, Filip De Somer F, Inge Everaert I, Wim Derave W, Caroline Van den Broecke C, Manon Huizing M, Nezahat Bostan N, Marijn Speeckaert M, Nico Callewaert N, Elisabeth Van Aken E, and Joris R. Delanghe JR. A Potential Role for Fructosamine-3-Kinase in Cataract Treatment Sander De Bruyne Int. J. Mol. Sci. 2021, 22, 3841. https://doi.org/10.3390/ ijms22083841

16. Roe, J.H. A colorimetric method for the determination of fructose in blood and urine. J. Biol. Chem. 1934 107:15-22

17. Wang K, Pierscionek BK. Biomechanics of the human lens and accommodative system: Functional relevance to physiological states. Prog Ret Eye Res 2019 71:114-131

18. Fernandez, D.C.; Bhargava, R.; Hewitt, S.M.; Levin, I.W. Infrared spectroscopic imaging for histopathologic recognition. Nat. Biotechnol. 2005, 23, 469-474

19. Varma, V.K.; Kajdacsy-Balla, A.; Akkina, S.K.; Setty, S.; Walsh, M.J. A label-free approach by infrared spectroscopic imaging for interrogating the biochemistry of diabetic nephropathy progression. Kidney Int. 2016, 89, 1153-1159

20. De Bruyne, S.; Speeckaert, M.M.; Delanghe, J.R. Applications of mid-infrared spectroscopy in the clinical laboratory setting. Crit. Rev. Clin. Lab. Sci. 2018, 55, 1-20

21. De Bruyne, S.; Speeckaert, R.; Boelens, J.; Hayette, M.-P.P.M.-P.; Speeckaert, M.; Delanghe, J. Infrared spectroscopy as a novel tool to diagnose onychomycosis. Br. J. Dermatol. 2019, 180, 637-646

**Claims**

1. A composition comprising a fructosyl amino oxidase for use to treat presbyopia, cataract, age-related macular degeneration, dry eye syndrome, diabetic retinopathy and/or glaucoma.

2. A composition for use according to claim 1 further comprising flavin adenine dinucleotide (FAD).

3. A composition for use according to claims 1or 2 further comprising a fructosamine-3-kinase and adenosine tri phosphate (ATP)

4. A composition for use according to claim 3 which further comprises magnesium ions.

5. A composition for use according to claims 1-4 which further comprises a peroxidase.

6. A composition for use according to claim 1-5 wherein said composition is administered as drops or via any other external application, or via an internal application.

**Patentansprüche**

1. Zusammensetzung, umfassend eine Fructosylaminooxidase zur Verwendung bei der Behandlung von Presbyopie, Katarakt, altersbedingter Makuladegeneration, Syndrom des trockenen Auges, diabetischer Retinopathie und/oder Glaukom.

2. Zusammensetzung zur Verwendung nach Anspruch 1, ferner umfassend Flavinadenindinukleotid (FAD).

3. Zusammensetzung zur Verwendung nach Anspruch 1 oder 2, ferner umfassend eine Fructosamin-3-Kinase und Adenosintriphosphat (ATP).

4. Zusammensetzung zur Verwendung nach Anspruch 3, die ferner Magnesiumionen umfasst.

5. Zusammensetzung zur Verwendung nach den Ansprüchen 1-4, die ferner eine Peroxidase umfasst.

6. Zusammensetzung zur Verwendung nach den Ansprüchen 1-5, wobei die Zusammensetzung in Form von Tropfen oder über eine andere externe Anwendung oder über eine interne Anwendung verabreicht wird.

**Revendications**

1. Composition comprenant une fructosyl amino oxydase pour une utilisation dans le traitement de la presbytie, de la cataracte, de la dégénérescence maculaire liée à l'âge, du syndrome de l'œil sec, de la rétinopathie diabétique et/ou du glaucome.

2. Composition pour une utilisation selon la revendication 1 comprenant en outre un dinucléotide flavine adénine (FAD).

3. Composition pour une utilisation selon la revendication 1 ou 2 comprenant en outre une fructosamine-3-kinase et de l'adénosine triphosphate (ATP).

4. Composition pour une utilisation selon la revendication 3 qui comprend en outre des ions magnésium.

5. Composition pour une utilisation selon les revendications 1 à 4 qui comprend en outre une peroxydase.

6. Composition pour une utilisation selon la revendication 1 à 5, ladite composition étant administrée comme gouttes ou via une quelconque autre application externe, ou via une application interne.

Fig 1A

Fig 1B

Fig 1C

Fig 1D

Fig 2

Fig 3A

Fig 3B

Fig 3C

Fig 3D

Fig 4

Fig 5A

Fig 5B

Fig 6

Fig 7A

Fig 7B

Fig 8

Fig 9

Fig 10

Fig 11

Fig 12

Fig 13

Fig 14

Fig 15

Fig 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2019149648 A
- WO 2020053188 A
- US 20050014935 A
- WO 20200053188 A

### Non-patent literature cited in the description

- **CAPUANO et al.** *J. Agric. Food Chem,* 2007, 4189
- **SAMBROOK et al.** Molecular Cloning: A laboratory Manual. Cold Spring Harbor press, 2012
- **AUSUBEL et al.** Current Protocols in Molecular Biology (supplement 114). John Wiley & Sons, 2016
- **AVERY NC et al.** The effects of the Maillard reaction on the physical properties and cell interactions of collagen. *Pathologic Biologic,* 2006, vol. 54, 387-95
- **NAGARAJ RH ; LINETSKY M ; STITT AW.** The pathogenic role of Maillard reaction in the eye. *Amino Acids.,* 2012, vol. 42, 1205-1220
- **ALVES M ; CALEGARI VC ; CUNHA DA ; SAAS MJA ; VELLOSO LA ; ROCHA EM.** Increased expression of advanced glycation end-products and their receptor, and activation of nuclear factor kappa-B in lacrimal glands of diabetic rats. *Diabetologia,* 2005, vol. 48, 2675-2681
- **SADOWSKA-BARTOSZ I ; BARTOSZ G.** *Effect of glycation inhibitors on aging and age-related diseases,* 2016, vol. 160, 1-18
- **BEJARANO E ; TAYLOR A.** *Too sweet: Problems of protein glycation in the eye. Exp Eye Res.,* 2019, vol. 178, 255-262
- **CHELONI R ; GANDOLFI SA ; SIGNORELLI C ; ODONE A.** Global prevalence of diabetic retinopathy: protocol for a systematic review and meta-analysis. *BMJ Open,* 2019, vol. 9, e022188
- **FERRI S ; KIM S ; TSUGAWA W ; SODE K.** Review of fructosyl amino acid oxidase engineering research; a glimpse into the future of hemoglobin A1c biosensing. *J Diabetes science and technology,* 2009, vol. 3, 585-592
- **LIN Z ; ZHENG J.** Occurrence, characteristics, and applications of fructosyl amine oxidases (amadoriases). *Appl Microbiol Biotechnol,* 2010, vol. 86, 1613-1619
- **SHEN et al.** *Abstract B44 of 2013 AACC Annual Meeting*
- **SAKAUE R ; HIRUMA M ; KAJIYAMA N ; KOYAMA Y.** Cloning and expression of fructosyl-amino acid oxidase gene grom Corynebacterium sp. 2-4-1 in Escherichia coli. *Biosci Biotechnol Biochem,* 2002, vol. 66, 1256-61
- **GOTE V ; SIKDER S ; SICOTTE J ; PAL D.** Ocular drug delivery: present innovations and future challenges. *J Pharmacol Exp Ther,* 2019, vol. 370, 602-624
- **TESSIER F ; OBRENOVICH M ; MONNIER VM.** Structure and mechanism of formation of human lens fluorophore LM-1. Relationship to vesperlysine A and the advanced Maillard reaction in aging, diabetes and cataractogenesis. *J Biol Chem,* 1999, vol. 274, 20796-20804
- **RAJAN M ; BEEDU SR.** Spectroscopic and biochemical correlations during the course of human lens aging. *BMC Ophthalmology,* 2006, vol. 6, 10
- **DE BRUYNE S ; LOES VAN SCHIE L ; JONAS HIMPE J ; FILIP DE SOMER F ; INGE EVERAERT I ; WIM DERAVE W ; CAROLINE VAN DEN BROECKE C ; MANON HUIZING M ; NEZAHAT BOSTAN N ; MARIJN SPEECKAERT M.** A Potential Role for Fructosamine-3-Kinase in Cataract Treatment Sander De Bruyne Int. *J. Mol. Sci.,* 2021, vol. 22, 3841, https://doi.org/10.3390/ ijms22083841
- **ROE, J.H.** A colorimetric method for the determination of fructose in blood and urine. *J. Biol. Chem.,* 1934, vol. 107, 15-22
- **WANG K ; PIERSCIONEK BK.** Biomechanics of the human lens and accommodative system: Functional relevance to physiological states. *Prog Ret Eye Res,* 2019, vol. 71, 114-131
- **FERNANDEZ, D.C ; BHARGAVA, R ; HEWITT, S.M ; LEVIN, I.W.** Infrared spectroscopic imaging for histopathologic recognition. *Nat. Biotechnol.,* 2005, vol. 23, 469-474

- **VARMA, V.K ; KAJDACSY-BALLA, A ; AKKINA, S.K ; SETTY, S. ; WALSH, M.J.** A label-free approach by infrared spectroscopic imaging for interrogating the biochemistry of diabetic nephropathy progression. *Kidney Int.,* 2016, vol. 89, 1153-1159

- **DE BRUYNE, S. ; SPEECKAERT, M.M ; DELANGHE, J.R.** Applications of mid-infrared spectroscopy in the clinical laboratory setting. *Crit. Rev. Clin. Lab. Sci.,* 2018, vol. 55, 1-20
- **DE BRUYNE, S. ; SPEECKAERT, R ; BOELENS, J. ; HAYETTE, M.-P.P.M.-P ; SPEECKAERT, M ; DELANGHE, J.** Infrared spectroscopy as a novel tool to diagnose onychomycosis. *Br. J. Dermatol.,* 2019, vol. 180, 637-646